# EUROPEAN PATENT APPLICATION

(11) **EP 3 804 720 A1**
(43) Date of publication of application: **14.04.2021**
(21) Application number: 19819975.4
(22) Date of filing: 11.06.2019
(51) Int. Cl.: A61K 31/454, A61K 9/16, A61K 9/20, A61K 9/48, A61K 47/20, A61K 47/26, A61K 47/32, A61K 47/38, A61P 31/04, A61P 31/06

(54) **DELAMANID-CONTAINING COMPOSITION**

(30) Priority: 11.06.2018 JP 2018111464
(71) Applicant: Otsuka Pharmaceutical Co., Ltd., Tokyo 101-8535 (JP)
(72) Inventor: NAKAMURA, Atsuya, Osaka-shi, Osaka 540-0021 (JP); YAMAZAKI, Hiroyuki, Osaka-shi, Osaka 540-0021 (JP); HASEGAWA, Masahiro, Osaka-shi, Osaka 540-0021 (JP); KAMADA, Naoki, Osaka-shi, Osaka 540-0021 (JP)
(74) Representative: Hoffmann Eitle
(86) International application number: PCT/JP2019/023006
(87) International publication number: WO 2019/240104

(57) **Abstract**

Provided is a composition comprising delamanid particles for which the formation of secondary particles is suppressed. Specifically, provided is a composition comprising (A) delamanid particles and (B) a surface stabilizer.

## Description

### Technical Field

The present invention relates to a composition comprising delamanid, a method for making the composition, and the like. All of the documents described in the present specification are incorporated herein by reference.

### Background Art

Delamanid is known to have excellent bactericidal action against *Mycobacterium tuberculosis,* multidrug-resistant *Mycobacterium tuberculosis,* and atypical mycobacteria (e.g., PTL 1 to PTL 3).

### Citation List

### Patent Literature

PTL 1: JP2004-149527A
PTL 2: WO2005/042542
PTL 3: WO2007/013477
PTL 4: WO2007/052738
PTL 5: JPH04-295420A
PTL 6: WO2002/024163
PTL 7: WO2004/041250

### Summary of Invention

### Technical Problem

In currently marketed delamanid formulations, the solubility of delamanid is improved and drug absorption is enhanced by dissolving delamanid together with a polymer in an organic solvent, followed by spray drying. However, in this method, the treatment of the organic solvent is very costly, and solvent recovery is required, which causes problems such as environmental burden.

The present inventors studied a method for preparing fine particles of delamanid in a manner that was as inexpensive as possible and that had a low environmental burden. A first attempt was made to increase the specific surface area by milling delamanid in water until it became fine particles (submicron particles). However, the submicron particles easily aggregated due to the aggregation force (e.g., intermolecular force, capillary force, and electrostatic force) acting after milling to produce secondary particles. Accordingly, it was not easy to efficiently mill delamanid and to prevent aggregation after milling. In addition, delamanid was extremely hydrophobic and thus difficult to mill in water.

### Solution to Problem

The present inventors found that when delamanid and a specific surface stabilizer were mixed and subjected to wet milling, it was possible to efficiently mill delamanid into submicron particles and also to prevent the formation of secondary particles. The present invention has been completed upon further improvement.

The present invention includes, for example, subject matters described in the following items.
Item 1. A composition comprising:
   (A) delamanid particles, and
   (B) a surface stabilizer.
Item 2. A composition comprising:
   (A) delamanid particles,
   (B-1) a polymer, and
   (B-2) a surfactant.
Item 3. The composition according to Item 2, comprising at least hydroxypropyl cellulose as polymer (B-1).
Item 4. The composition according to Item 2 or 3, comprising at least a nonionic surfactant and/or an anionic surfactant as surfactant (B-2).
Item 5. The composition according to any one of Items 2 to 4, comprising (A) delamanid particles, (B-1) hydroxypropyl cellulose, and (B-2) a sucrose fatty acid ester (preferably at least one member selected from the group consisting of sucrose laurate ester, sucrose myristate ester, sucrose palmitate ester, sucrose stearate ester, sucrose oleate ester, sucrose behenate ester, and sucrose erucate ester; and more preferably at least one member selected from the group consisting of sucrose stearate ester and sucrose oleate ester) and/or a diester of alkyl alcohol and sulfosuccinic acid or a salt thereof (preferably a diester of linear or branched C₈₋₁₈ alkyl alcohol and sulfosuccinic acid or a salt thereof, where the two alkyl alcohols may be the same or different, and are preferably the same; the diester or a salt thereof is particularly preferably dioctyl sodium sulfosuccinate).
Item 6. The composition according to any one of Items 2 to 5, comprising 2 to 20 parts by mass of component (B-1) and 2 to 55 parts by mass of component (B-2) based on 100 parts by mass of component (A).
Item 7. The composition according to Item 6, comprising 2 to 15 parts by mass of sucrose fatty acid ester and 2 to 40 parts by mass of dioctyl sodium sulfosuccinate based on 100 parts by mass of component (A).
Item 8. The composition according to any one of Items 2 to 7, further comprising sugar and/or sugar alcohol (preferably mannitol) (preferably in an amount of 30 to 200 parts by mass based on 100 parts by mass of component (A)).
Item 9. The composition according to Item 8, comprising 2 to 20 parts by mass of component (B-1), 2 to 55 parts by mass of component (B-2), and 30 to 200 parts by mass of sugar and/or sugar alcohol, based on 100 parts by mass of component (A).
Item 10. The composition according to Item 8 or 9, comprising 2 to 15 parts by mass of sucrose fatty acid ester, 2 to 40 parts by mass of dioctyl sodium sulfosuccinate, and 30 to 200 parts by mass of mannitol, based on 100 parts by mass of component (A).
Item 11. The composition according to any one of Items 1 to 10, which is a granular composition.
Item 12. The composition according to any one of Items 1 to 11, wherein delamanid particles (A) have an average particle size of 350 nm or less.
Item 13. A pharmaceutical oral solid formulation obtainable from the granular composition according to Item 11 or 12.
Item 14. The pharmaceutical oral solid formulation according to Item 13, which is a tablet or capsule.

### Advantageous Effects of Invention

Since the composition comprising delamanid particles included in the present invention comprises a specific surface stabilizer, the formation of secondary particles of the delamanid particles during administration is suppressed (the dispersibility in solvents being particularly excellent), and the dissolution rate is improved. Further, according to the method for making a composition comprising delamanid particles included in the present invention, delamanid can be efficiently milled, and the formation of secondary particles of the resulting delamanid can be suppressed.

### Brief Description of Drawings

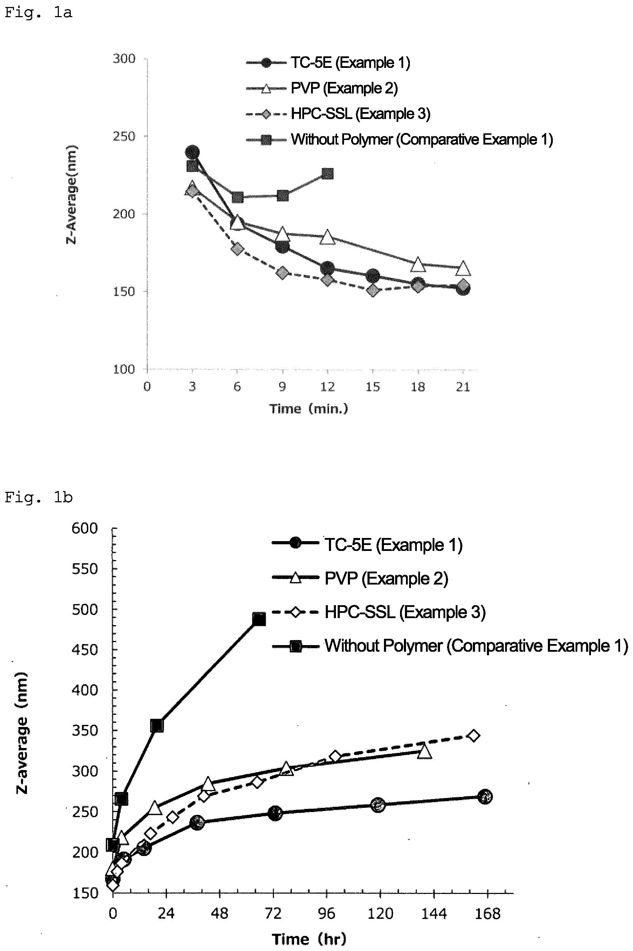

Fig. 1a shows the results of measuring the change in the average particle size of delamanid particles every 3 minutes from the start of milling, when compositions (suspensions) comprising delamanid particles, a polymer, dioctyl sodium sulfosuccinate (DOSS), and a sucrose fatty acid ester were prepared by wet milling.
Fig. 1b shows the results of measuring the change in the average particle size of delamanid particles when the delamanid particle-containing suspensions prepared by wet milling shown in Fig. 1a were stored for several days.

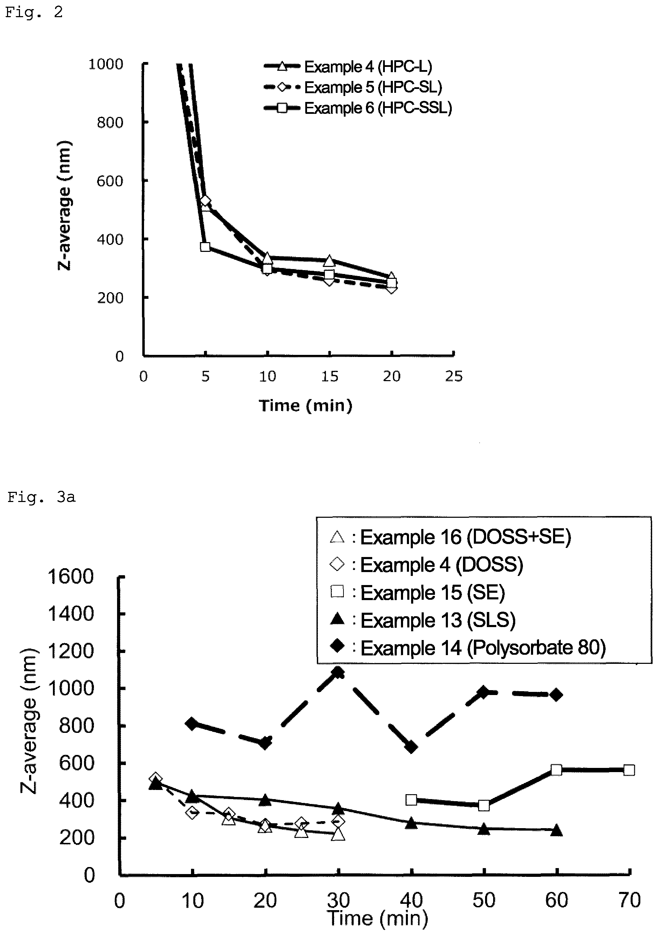

Fig. 2 shows the results of measuring the change in the average particle size of delamanid particles every 5 minutes from the start of milling, when compositions (suspensions) comprising delamanid particles, hydroxypropyl cellulose, and dioctyl sodium sulfosuccinate (DOSS) were prepared by wet milling.
Fig. 3a shows the results of measuring the change in the average particle size (Z-average) of delamanid particles when compositions (suspensions) comprising delamanid particles, hydroxypropyl cellulose, and various surfactants were prepared by wet milling.

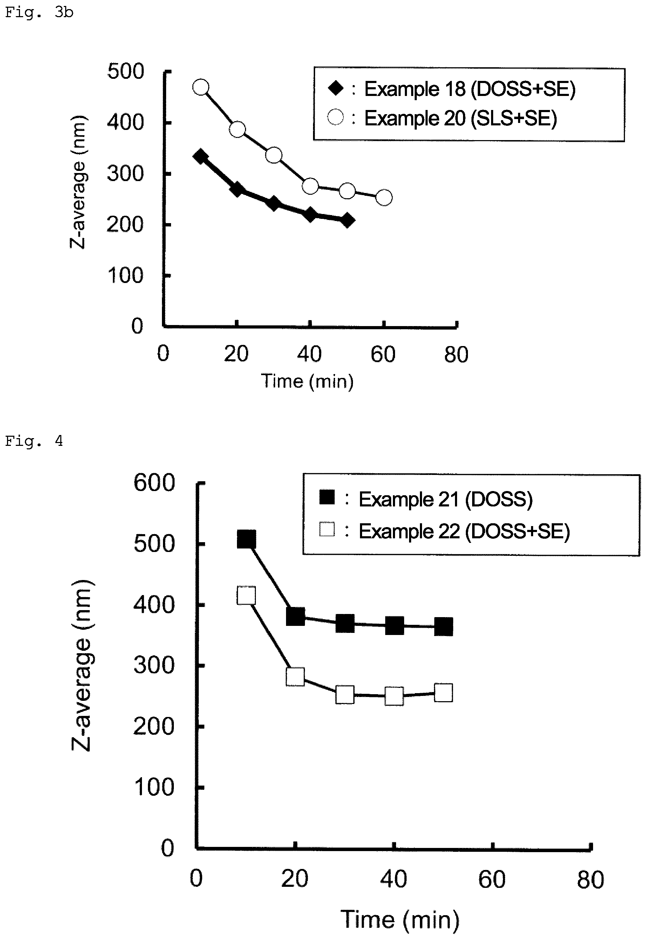

Fig. 3b shows the results of measuring the change in the average particle size (Z-average) of delamanid particles when compositions (suspensions) comprising delamanid particles, hydroxypropyl cellulose, and a sucrose fatty acid ester, and further comprising dioctyl sodium sulfosuccinate (DOSS) or sodium dodecyl sulfate (SLS) were prepared by wet milling.
Fig. 4 shows the results of measuring the change in the average particle size (Z-average) of delamanid particles when compositions (suspensions) comprising delamanid particles, hydroxypropyl cellulose, and dioctyl sodium sulfosuccinate (DOSS), and further comprising or not comprising a sucrose fatty acid ester were prepared by wet milling.

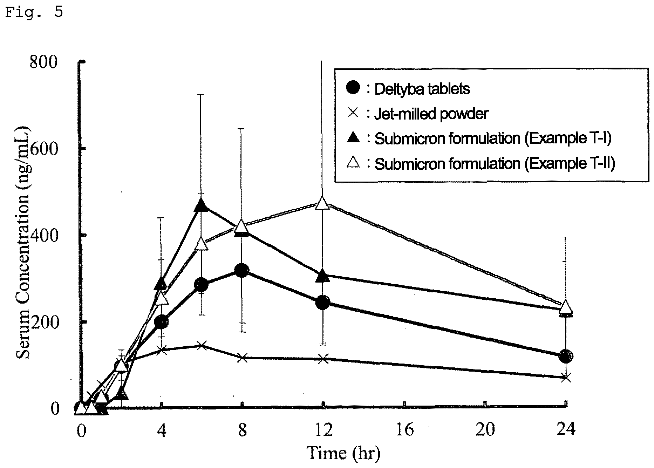

Fig. 5 shows the change in the serum concentration when submicron powders comprising delamanid particles were administered to dogs.

### Description of Embodiments

Embodiments of the present invention are described in more detail below.

The composition comprising delamanid particles included in the present invention comprises (A) delamanid particles and (B) a surface stabilizer (hereinafter, this composition is also referred to as "the composition of the present invention"). Hereinafter, the delamanid particles are also referred to as "component (A)," and the surface stabilizer is also referred to as "component (B)."

Delamanid is a compound represented by the following formula:

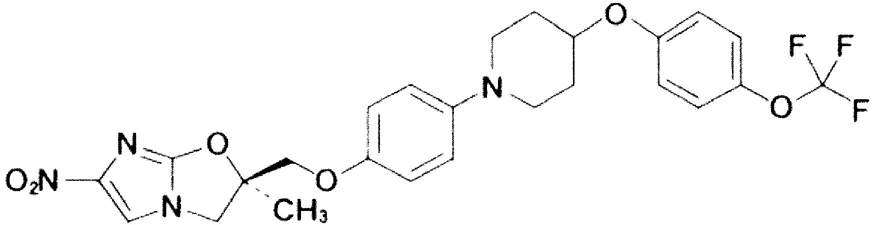

As described above, delamanid is known to have excellent bactericidal action against *Mycobacterium tuberculosis,* multidrug-resistant *Mycobacterium tuberculosis,* and atypical mycobacteria. Further, delamanid can be produced by a known method or a method that can be easily conceived of from a known method.

Examples of surface stabilizers include known organic and inorganic pharmaceutical excipients. Such excipients include various polymers, low-molecular-weight oligomers, natural products, and surfactants. Surfactants include nonionic and ionic surfactants, and ionic surfactants include amphoteric, anionic, and cationic surfactants. Surface stabilizers can be used alone or in combination of two or more.

More specifically, examples of surface stabilizers include hydroxypropyl methylcellulose (also called "hypromellose"), hydroxypropyl cellulose, polyvinylpyrrolidone, sodium lauryl sulfate, dioctyl sodium sulfosuccinate (DOSS), gelatin, casein, lecithin (phosphatide), dextran, gum arabic, cholesterol, tragacanth, stearic acid, benzalkonium chloride, calcium stearate, glycerol monostearate, cetostearyl alcohol, sucrose fatty acid esters, sorbitan esters, polyvinyl caprolactam-polyvinyl acetate-polyethylene glycol graft polymers (e.g., Solplus (registered trademark)), polyoxyethylene alkyl ethers (e.g., Cetomacrogol 1000), polyoxyethylene castor oil derivatives, polyoxyethylene sorbitan fatty acid esters (e.g., commercially available Tween (registered trademark) series, such as Tween 20 (registered trademark) and Tween 80 (registered trademark) (ICI Speciality Chemicals)); polyethylene glycols (e.g., Carbowaxs 3550 (registered trademark) and 934 (registered trademark) (Union Carbide)), polyoxyethylene stearate, colloidal silicon dioxide, phosphates, phosphate esters, calcium carboxymethyl cellulose, sodium carboxymethyl cellulose, methyl cellulose, hydroxyethyl cellulose, hypromellose phthalate, amorphous cellulose, magnesium aluminum silicate, triethanolamine, polyvinyl alcohol (PVA), 4-(1,1,3,3-tetramethylbutyl)-phenol polymer with ethylene oxide and formaldehyde (also known as tyloxapol, sperion, and triton), poloxamers (e.g., Pluronics F68 (registered trademark) and F108 (registered trademark), which are block copolymers of ethylene oxide and propylene oxide); poloxamines (e.g., Tetronic 908 (registered trademark), also known as Poloxamine 908 (registered trademark); this is a tetrafunctional block copolymer obtained by sequentially adding propylene oxide and ethylene oxide to ethylenediamine (BASF Wyandotte Corporation, Parsippany, N.J.)); Tetronic 1508 (registered trademark) (T-1508) (BASF Wyandotte Corporation), Triton X-200 (registered trademark) (Rohm and Haas), which is alkylaryl polyester sulfonate; Crodesta F-110 (registered trademark) (Croda Inc.), which is a mixture of sucrose stearate and sucrose distearate; p-isononylphenoxypoly-(glycidol), which is also known as Olin-lOG (registered trademark) or Surfactant 10-G (registered trademark) (Olin Chemicals, Stamford, CT); Crodesta SL-40 (registered trademark) (Croda, Inc.); SA9OHCO (Eastman Kodak Co.), which is C₁₈H₃₇CH₂(CON(CH₃)-CH₂(CHOH)₄(CH₂OH)₂; decanoyl-N-methylglucamide; n-decyl-β-D-glucopyranoside; n-decyl-β-D-maltopyranoside; n-dodecyl-β-D-glucopyranoside; n-dodecyl-β-D-maltoside; heptanoyl-N-methylglucamide; n-heptyl-β-D-glucopyranoside; n-heptyl-β-D-thioglucoside; n-hexyl-β-D-glucopyranoside; nonanoyl-N-methylglucamide; n-noyl-β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivatives, PEG-vitamin A, PEG-vitamin E, lysozyme, random copolymers of vinyl pyrrolidone and vinyl acetate; and the like.

Examples of useful cationic surface stabilizers include, but are not limited to, cationic polymers, cationic biopolymers, cationic polysaccharides, cationic cellulosic materials, alginates, phospholipids, and cationic non-polymeric compounds, such as zwitterionic stabilizers, poly-n-methylpyridinium, anthryul pyridinium chloride, cationic phospholipids, chitosan, polylysine, polyvinyl imidazole, polybrene, polymethylmethacrylate trimethylammonium bromide (PMMTMABr), hexyldesyltrimethylammonium bromide (HDMAB), and polyvinylpyrrolidone-2-dimethylaminoethyl methacrylate dimethyl sulfate.

Other examples of useful cationic surface stabilizers include, but are not limited to, cationic lipids, sulfonium, phosphonium, and quaternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅ dimethyl hydroxyethyl ammonium chloride or bromide, coconut dimethyl hydroxyethyl ammonium chloride or bromide, myristyl trimethyl ammonium methyl sulfate, lauryl dimethyl benzyl ammonium chloride or bromide, lauryl dimethyl (ethenoxy)₄ ammonium chloride or bromide, N-alkyl (C₁₂₋₁₈) dimethylbenzyl ammonium chloride, N-alkyl (C₁₄-₁₈) dimethyl-benzyl ammonium chloride, N-tetradecylidmethylbenzyl ammonium chloride monohydrate, dimethyl didecyl ammonium chloride, N-alkyl (C₁₂₋₁₄) dimethyl 1-naphthylmethyl ammonium chloride, trimethylammonium halide, alkyltrimethylammonium salts and dialkyl-dimethylammonium salts, lauryl trimethyl ammonium chloride, ethoxylated alkyamidoalkyldialkylammonium salt and/or ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl (C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂,C₁₅,C₁₇ trimethyl ammonium bromide, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyltrioctylammonium chloride (ALIQUAT 336 (trademark)), POLYQUAT 10 (trademark), tetrabutylammonium bromide, benzyltrimethylammonium bromide, choline esters (e.g., choline esters of fatty acids), benzalkonium chloride, stearalkonium chloride compounds (e.g., stearyltrimonium chloride and di-stearyldimonium chloride), cetyl pyridinium bromide or chloride, halide salts of quaternized polyoxyethylalkylamines, MIRAPOL (trademark) and ALKAQUAT (trademark) (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine; amine salts, such as lauryl amine acetate, stearyl amine acetate, alkylpyridinium salt, and alkylimidazolium salt; amine oxides; imide azolinium salts; protonated quaternary acrylamides; methylated quaternary polymers, such as poly[dialkyl dimethyl ammonium chloride] and poly-[N-methylvinylpyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry (Marcel Dekker, 1990).

Examples of non-polymeric surface stabilizers include any non-polymeric compounds, such as benzalkonium chloride, carbonium compounds, phosphonium compounds, oxonium compounds, halonium compounds, cationic organometallic compounds, quaternary phosphorus compounds, pyridinium compounds, anilinium compounds, ammonium compounds, hydroxyl ammonium compounds, primary ammonium compounds, secondary ammonium compounds, tertiary ammonium compounds, and quaternary ammonium compounds of the formula NR₁R₂R₃R₄ (+). In the compounds of the formula NR₁R₂R₃R₄⁽⁺⁾,
(i) none of R₁ to R₄ is CH₃,
(ii) one of R₁ to R₄ is CH₃,
(iii) three of R₁ to R₄ are CH₃,
(iv) all of R₁ to R₄ are CH₃,
(v) two of R₁ to R₄ are CH₃, one of R₁ to R₄ is C₆H₅CH₂, and one of R₁ to R₄ is an alkyl chain having 7 or fewer carbon atoms,
(vi) two of R₁ to R₄ are CH₃, one of R₁ to R₄ is C₆H₅CH₂, and one of R₁ to R₄ is an alkyl chain having 19 or more carbon atoms,
(vii) two of R₁ to R₄ are CH₃, and one of R₁ to R₄ is a group C₆H₅(CH₂)ₙ, provided that n > 1,
(viii) two of R₁ to R₄ are CH₃, one of R₁ to R₄ is C₆H₅CH₂, and one of R₁ to R₄ contains at least one heteroatom,
(ix) two of R₁ to R₄ are CH₃, one of R₁ to R₄ is C₆H₅CH₂, and one of R₁ to R₄ contains at least one halogen,
(x) two of R₁ to R₄ are CH₃, one of R₁ to R₄ is C₆H₅CH₂, and one of R₁ to R₄ contains at least one ring fragment,
(xi) two of R₁ to R₄ are CH₃, and one of R₁ to R₄ is a phenyl ring, or
(xii) two of R₁ to R₄ are CH₃, and two of R₁ to R₄ can be purely aliphatic fragments.

Examples of such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cetylamine hydrofluoride, chloroallyl methenamine chloride (Quaternium-15), distearyldimonium chloride (Quaternium-5), dodecyl dimethyl ethylbenzyl ammonium chloride (Quaternium-14), Quaternium-22, Quaternium-26, Quaternium-18 hectorite, dimethylaminoethyl chloride hydrochloride, cysteine hydrochloride, diethanolamine POE(10) oleyl ether phosphate, diethanolammonium POE(3) oleyl ether phosphate, tallow alkonium chloride, dimethyldioctadecyl ammonium bentonite, stearalkonium chloride, domiphen bromide, denatonium benzoate, miristalkonium chloride, laurtrimonium chloride, ethylenediamine dihydrochloride, guanidine hydrochloride, pyridoxine HC1, iofetamine hydrochloride, meglumine hydrochloride, methylbenzethonium chloride, myrtrimonium bromide, oleyltrimonium chloride, polyquaternium-1, procaine hydrochloride, cocobetaine, stearalkonium bentonite, stearalkonium hectorite, stearyl trihydroxyethyl propylenediamine dihydrofluoride, tallow trimonium chloride, and hexadecyltrimethylammonium bromide.

The composition of the present invention preferably comprises at least one polymer and at least one surfactant as surface stabilizers. Particularly from the viewpoint of dispersibility, the polymer is preferably hydroxypropyl cellulose and/or hydroxypropyl methylcellulose. Further, the surfactant is preferably a nonionic surfactant and/or an anionic surfactant. The nonionic surfactant is particularly preferably a sucrose fatty acid ester. The anionic surfactant is particularly preferably a diester of alkyl alcohol and sulfosuccinic acid or a salt thereof. Polymers can be used alone or in combination of two or more, and surfactants can also be used alone or in combination of two or more. Hereinafter, the polymer is also referred to as "component (B-1)," and the surfactant is also referred to as "component (B-2)." Component (B-1) is preferably contained, for example, in an amount of 2 to 25 parts by mass based on 100 parts by mass of component (A). Further, component (B-2) is preferably contained, for example, in an amount of 2 to 55 parts by mass based on 100 parts by mass of component (A).

The hydroxypropyl cellulose is preferably one having a molecular weight of about 20000 to 200000, more preferably about 25000 to 175000, even more preferably about 30000 to 150000, and still even more preferably about 40000 to 140000. The upper limit of the molecular weight may be about 130000, 120000, 110000, 100000, 90000, 80000, 70000, 60000, or 50000. The molecular weight is a value obtained by the GPC method.

Further, the hydroxypropyl cellulose is preferably one having a viscosity (mPa·s) of 20°C/2% aqueous solution of about 2 to 10. For example, this viscosity is preferably about 6 to 10, about 3 to 5.9, or about 2 to 2.9.

The sucrose fatty acid ester is an ester of sucrose and fatty acid. Specific examples of the fatty acid include lauric acid, myristic acid, palmitic acid, stearic acid, oleic acid, behenic acid, erucic acid, and the like. Sucrose stearate ester and sucrose oleate ester are particularly preferable.

About 30% or more of the sucrose fatty acid ester used is preferably a sucrose fatty acid monoester, and about 50% or more, about 55% or more, about 60% or more, about 65% or more, about 70% or more, or about 75% or more thereof is more preferably a sucrose fatty acid monoester. The monoester bond of the sucrose fatty acid monoester is preferably formed by the OH group underlined in the structural formula of sucrose shown below and the COOH group of the fatty acid.

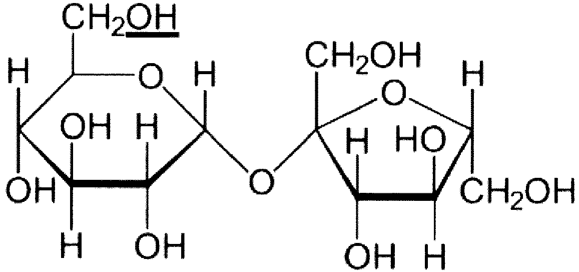

As the alkyl alcohols that constitute the diester of alkyl alcohol and sulfosuccinic acid, linear or branched alkyl alcohols having 8 to 18 (8, 9, 10, 11, 12, 13, 14, 15, 16, 17, or 18) carbon atoms are preferred. The alkyl alcohols are preferably alkyl monoalcohols, and more preferably n-alkyl monoalcohols. The two alkyl alcohols constituting the diester may be the same or different, and are preferably the same.

Examples of salts of the diester of alkyl alcohol and sulfosuccinic acid include sodium salts and potassium salts, but are not particularly limited thereto.

A particularly preferred example of the diester or a salt thereof is dioctyl sodium sulfosuccinate.

When the composition of the present invention contains a salt of the diester, ionization may occur, depending on the form of the composition (e.g., in the form of a suspension); however, in the present specification, even in such a case, the salt of the diester is contained in the composition of the present invention.

Hydroxypropyl celluloses can be used alone or in combination of two or more, and sucrose fatty acid esters can also be used alone or in combination of two or more.

When the composition of the present invention comprises components (A), (B-1), and (B-2), in a preferred embodiment, the composition of the present invention comprises 2 to 20 parts by mass of component (B-1) and 2 to 55 parts by mass of component (B-2) when the amount of component (A) is 100 parts by mass. The lower limit of the range of component (B-1) may be, for example, 2.5, 3, 3.5, or 4 parts by mass. Further, the upper limit of the range of component (B-1) may be, for example, 19.5, 19, 18.5, 18, 17.5, 17, 16.5, 16, 15.5, 15, 14.5, 14, 13.5, 13, 12.5, 12, 11.5, 11, 10.5, 10, 9.5, 9, 8.5, 8, 7.5, 7, 6.5, or 6 parts by mass. The lower limit of the range of component (B-2) may be, for example, 2.5, 3, 3.5, 4, 4.5, 5, 5.5, 6, 6.5, or 7 parts by mass, or may be 30, 29, 28, 27, 26, 25, 24, 23, 22, 21, 20, 19, 18, 17, 16, 15, 14, 13, 12, 11, 10, 9, or 8 parts by mass. Further, the upper limit of the range of component (B-2) may be, for example, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, or 8 parts by mass.

The composition of the present invention comprising 2 to 20 parts by mass of component (B-1) and 2 to 55 parts by mass of component (B-2) when the amount of component (A) is 100 parts by mass is also particularly referred to as "composition α." A particularly preferred example of composition α is a composition comprising 2 to 20 parts by mass or 2 to 15 parts by mass of sucrose fatty acid ester, and 2 to 40 parts by mass, 2 to 35 parts by mass, 2 to 25 parts by mass, 2 to 20 parts by mass, or 2 to 15 parts by mass, of dioctyl sodium sulfosuccinate, when the amount of component (A) is 100 parts by mass.

The composition of the present invention may further comprise sugar and/or sugar alcohol. Examples of sugars and sugar alcohols include mannitol, trehalose, xylitol, erythritol, lactose sucrose, dextrin, and the like. Among these, mannitol (particularly D-mannitol) is preferable. Sugars and sugar alcohols can be used alone or in combination of two or more.

When the composition of the present invention comprises sugar and/or sugar alcohol, the content thereof is preferably 30 to 200 parts by mass, more preferably 30 to 150 parts by mass or 30 to 100 parts by mass, even more preferably 35 to 90 parts by mass, still even more preferably 40 to 80 parts by mass, and particularly preferably 45 to 75 parts by mass, based on 100 parts by mass of component (A).

When the composition of the present invention comprises component (A), component (B-1), component (B-2), and sugar and/or sugar alcohol, in a preferred embodiment, the composition of the present invention comprises 2 to 20 parts by mass of component (B-1), 2 to 55 parts by mass of component (B-2), and 30 to 200 parts by mass of sugar and/or sugar alcohol, when the amount of component (A) is 100 parts by mass. The lower limit of the range of component (B-1) may be, for example, 2.5, 3, 3.5, or 4 parts by mass. Further, the upper limit of the range of component (B-1) may be, for example, 19.5, 19, 18.5, 18, 17.5, 17, 16.5, 16, 15.5, 15, 14.5, 14, 13.5, 13, 12.5, 12, 11.5, 11, 10.5, 10, 9.5, 9, 8.5, 8, 7.5, 7, 6.5, or 6 parts by mass. The lower limit of the range of component (B-2) may be, for example, 2.5, 3, 3.5, 4, 4.5, 5, 5.5, 6, 6.5, or 7 parts by mass. Further, the range of component (B-2) may be, for example, about 2 to 20 parts by mass, or more than 20 parts by mass and 55 parts by mass or less. When the range of component (B-2) is about 2 to 20 parts by mass, the lower limit may be, for example, 2.5, 3, 3.5, 4, 4.5, 5, 5.5, 6, 6.5, or 7 parts by mass. Further, the upper limit may be, for example, 19, 18, 17, 16, 15, 14, 13, 12, 11, 10, 9, or 8 parts by mass. When the range of component (B-2) is more than 20 parts by mass and 55 parts by mass or less, it may be, for example, 20.5 to 55 parts by mass. Within this range, the lower limit may be, for example, 21, 21.5, 22, 22.5, 23, 23.5, 24, 24.5, 25, 25.5, 26, 26.5, 27, 27.5, 28, 28.5, or 29 parts by mass. Further, within this range, the upper limit may be, for example, 52.5, 50, 47.5, 45, 42.5, 40, 39.5, 39, 38.5, 38, 37.5, 37, 36.5, 36, 35.5, or 35 parts by mass. Moreover, the range of sugar and/or sugar alcohol is preferably 30 to 150 parts by mass or 30 to 100 parts by mass, more preferably 35 to 90 parts by mass, even more preferably 40 to 80 parts by mass, and particularly preferably 45 to 75 mass%. In particular, when 45 to 75 mass% thereof is contained, the lower limit may be, for example, 46, 47, 48, 49, 50, 51, 52, 53, 54, 55, 56, 57, 58, or 59 parts by mass. Further, the upper limit may be, for example, 74, 73, 72, 71, 70, 69, 68, 67, 66, 65, 64, 63, 62, or 61 parts by mass.

The composition of the present invention comprising 2 to 20 parts by mass of component (B-1), 2 to 20 parts by mass of component (B-2), and 30 to 200 parts by mass of sugar and/or sugar alcohol, when the amount of component (A) is 100 parts by mass, is also particularly referred to as "composition β." Further, the composition of the present invention comprising 2 to 20 parts by mass of component (B-1), more than 20 parts by mass and 55 parts by mass or less of component (B-2), and 30 to 200 parts by mass of sugar and/or sugar alcohol, when the amount of component (A) is 100 parts by mass, is also particularly referred to as "composition γ." Compositions β and γ can be regarded as specific compositions α further comprising 30 to 200 parts by mass of sugar and/or sugar alcohol based on 100 parts by mass of component (A). Particularly preferred examples of composition β include compositions comprising 2 to 15 parts by mass of sucrose fatty acid ester, 2 to 15 parts by mass of dioctyl sodium sulfosuccinate, and 30 to 200 parts by mass of mannitol, when the amount of component (A) is 100 parts by mass. In addition, particularly preferred examples of composition γ include compositions comprising 2 to 15 parts by mass of sucrose fatty acid ester, more than 15 parts by mass and 40 parts by mass or less of dioctyl sodium sulfosuccinate, and 30 to 200 parts by mass of mannitol, when the amount of component (A) is 100 parts by mass.

The description of compositions α, β, and γ only describes the preferred examples, and the composition of the present invention is not particularly limited thereto.

The form of the composition of the present invention is not particularly limited. For example, the composition of the present invention can be preferably used in the form of granules (particularly powder), suspensions, solids (particularly tablets), or the like. Further, the composition of the present invention can be prepared as each form by a known method or a method that can be easily conceived of from a known method.

For example, the composition of the present invention in the form of a suspension can be prepared by subjecting raw materials (preferably including at least delamanid, component (B-1), and component (B-2)) to wet milling together with water to mill delamanid. Further, for example, the composition of the present invention in the form of powder can be prepared by powdering the suspension. Examples of the powdering method include a spray-drying method, a wet granulation method (particularly fluidized bed granulation method), and the like. Moreover, for example, the composition of the present invention in the form of a solid can be prepared by solidifying (e.g., compressing) the obtained powder under pressure.

Further, for example, the granular composition can be directly inserted into capsules and used as capsules. Moreover, for example, tablets can be prepared by collecting and compressing the granular composition. Further, for example, a suspension can be prepared by dispersing the granular composition in water. These can be preferably used as pharmaceutical oral formulations (solid or liquid).

In addition, suspensions can be used as suspension agents, injections, drips, and the like. Other usable examples include various dosage forms, such as granules, pills, liquids, powders, spirits, syrups, and lozenges.

Examples of other components that may be combined include excipients, binders, fillers, lubricants, suspension agents, sweeteners, flavoring agents, preservatives, buffers, wetting agents, disintegrants, fluidizers, lubricants, coating agents, coloring agents, foaming agents, and the like. Other such components are known in the art.

Specific examples of some of the other components are provided below. Examples of excipients include lactose, anhydrous lactose, refined white sugar, white sugar, D-mannitol, D-sorbitol, xylitol, erythritol, dextrin, crystalline cellulose, microcrystalline cellulose, corn starch, potato starch, anhydrous calcium hydrogen phosphate, and the like. Examples of binders include hydroxypropyl cellulose, hydroxypropyl methylcellulose, polyvinylpyrrolidone, pregelatinized starch, syrup, starch syrup, and the like. Examples of disintegrants include sodium carboxymethyl starch, carmellose, carmellose calcium, carmellose sodium, croscarmellose sodium, crospovidone, low-substituted hydroxypropyl cellulose, partially pregelatinized starch, and the like. Examples of fluidizers include light anhydrous silicic acid, synthetic aluminum silicate, hydrous silicon dioxide, calcium stearate, magnesium aluminometasilicate, talc, and the like. Examples of lubricants include magnesium stearate, calcium stearate, magnesium silicate, magnesium oxide, talc, hardened oil, sucrose fatty acid ester, sodium stearyl fumarate, and the like. Examples of coating agents include hydroxypropyl methylcellulose, polyvinyl alcohol, polysorbate, macrogol, talc, and the like. Examples of sweeteners include aspartame, acesulfame potassium, sucralose, saccharin, saccharin sodium, starch syrup, fructose, and the like. Examples of flavoring agents include lemon, grapefruit, orange, cherry, strawberry, apple, pineapple, peach, yogurt, chocolate, matcha, and the like.

Further, for example, capsules can be prepared by inserting the granular composition into, for example, hard capsules or soft capsules. More specifically, capsules can be prepared, for example, by inserting the granular composition into hard capsules made of gelatin, hydroxypropyl methylcellulose, polyvinyl alcohol, or the like, or gelatin-based soft capsules. Alternatively, capsules can also be prepared by mixing the granular composition with various carriers mentioned above according to a known method, optionally further granulating and compressing the mixture, and inserting the resultant into the capsules described above.

Moreover, for example, an example of the composition of the present invention in the form of a suspension can be prepared by mixing delamanid and component (B) (preferably components (B-1) and (B-2)) with an appropriate amount of medium (preferably water), followed by wet milling. Delamanid is a known compound and can be prepared by a known method (e.g., the method disclosed in PTL 1, 2, or 3 mentioned above). Component (B) (preferably components (B-1) and (B-2)) is also a known substance and can be prepared by a known method. Commercially available products can be purchased and used.

The amount of medium (e.g., water or a known buffer) is not particularly limited as long as wet milling can be performed. For example, it is preferable to use about 350 to 2000 parts by mass of medium based on 100 parts by mass of component (A).

More specifically, for example, a suspension can be obtained by dispersing delamanid (preferably pre-milled in advance with a hammer mill etc.) and component (B) in an appropriate amount of water, and wet-milling the resultant with zirconia beads in a wet-milling machine (e.g., DYNO-MILL (Multi-Lab, Shinmaru Enterprises Corporation)). The milling time can be set appropriately. For example, milling can be performed until the average particle size of the delamanid particles hardly changes even after the milling treatment, or until the desired average particle size is obtained. For example, the milling time is about 5 to 90 minutes. Further, component (B-1) and/or component (B-2) may be added, if necessary, to the suspension after wet milling. Other components may also be added, if necessary. The post-added suspension thus obtained is also included in the composition of the present invention. In addition, it is also possible to change the form of the composition by a conventional method using the suspension (including the above suspension after wet milling and post-added suspension). The suspension can be directly used as the composition of the present invention, or the suspension can be formed into powder (granular composition). Such powder is also included in the composition of the present invention. Known methods such as spray drying, freeze drying, granulation (high shear granulation, fluidized bed granulation, etc.), drug layering, and extrusion granulation can be used as the method for obtaining powder from the suspension. When granulation is carried out using a method of attaching the suspension around a core material (e.g., fluidized bed granulation or drug layering), examples of the core material include, but are not particularly limited to, hydroxypropyl cellulose (preferably low-substituted hydroxypropyl cellulose), calcium silicate, mannitol, starch, and the like.

For example, the granular composition obtained by spray-drying the suspension may preferably have a structure in which surface stabilizer (B) is attached to the surface of delamanid particles (A). Due to the structure in which component (B) is attached to the surface of component (A) (particles), it is possible to efficiently suppress the aggregation of particles, for example, during wet milling or when the particles are dispersed or dissolved in a solution. For example, although it is not intended to limit the interpretation, it is considered that when polymer (B-1) is attached to the surface of component (A), the polymer can act as a steric hindrance that suppresses the aggregation of the particles. It is also considered that, for example, when surfactant (B-2) is attached to the surface of component (A), it can act as a repulsive due to the charge and/or steric hindrance in the solution, thereby efficiently suppressing the aggregation of the particles.

The average particle size of component (A) (i.e., delamanid particles) contained in the composition of the present invention is preferably submicron (e.g., less than 1000 nm, preferably 900 nm or less, and more preferably 800, 700, 600, 500, or 400 nm or less), particularly preferably 350 nm or less, and more preferably 340, 330, 320, 310, or 300 nm or less. The lower limit is not particularly limited, but is preferably 50 nm or more, and more preferably 60, 70, 80, 90, or 100 nm or more. This average particle size is the average particle size (Z-average) measured by dynamic light scattering. For example, when the composition of the present invention is in the form of powder, the average particle size can be measured by dispersing or dissolving the powder in a dispersion medium. Further, for example, when the composition of the present invention is in the form of a dispersion, it can be directly used for measurement. In the measurement of the average particle size by dynamic light scattering, if components other than component (A) (e.g., component (B)) are soluble in a dispersion medium (e.g., water or a buffer), it can be said that the obtained average particle size is substantially the average particle size of component (A) (particles). Therefore, when component (B) used in the granular composition of the present invention is soluble in water, and when components other than components (A) and (B) are not contained, or when components other than components (A) and (B) are also soluble in water, the average particle size of the granular composition of the present invention is substantially the average particle size of component (A).

When a composition comprising component (A), component (B-1), and component (B-2) is prepared by wet milling, the ratio of parts by mass of delamanid, component (B-1), and component (B-2) to be subjected to wet milling is set to a preferable ratio of parts by mass of components contained in the composition, whereby the composition of the present invention that satisfies the above preferable range of parts by mass ratio of components can be easily obtained. However, when a relatively large amount of surfactant (B-2) is used during wet milling, many bubbles may be generated, which makes milling difficult. When only a relatively small amount of surfactant (B-2) is used, the aggregation-suppressing effect may not be obtained during wet milling. On the other hand, when the granular composition of the present invention comprises a relatively larger amount of surfactant (B-2), the aggregation-suppressing effect is higher, and particularly when the composition is redispersed (resuspended), the aggregation-suppressing effect is higher. That is, wet milling is difficult when the amount of surfactant is relatively large or very small, whereas the aggregation-suppressing effect (particularly aggregation-suppressing effect during redispersion) is less likely to be obtained when there is no or very little surfactant. More specifically, for example, in the case of around the ratio of parts by mass of components of composition α or composition β, milling is not difficult even when all the components are subjected to wet milling; however, in the case of the ratio of parts by mass of components of composition γ, the ratio of part by mass of surfactant (B-2) is relatively high; thus, when all the components are subjected to wet millng, bubbles may be generated, thereby making milling difficult, which may reduce the milling efficiency. Moreover, when a large amount of water is used so that bubbles are not generated, milling may be inefficient, and the particle aggregation-suppressing effect may be reduced. Therefore, in such a case, wet milling is performed using a relatively small amount of surfactant (B-2), and a larger amount of surfactant (B-2) is added to the milled suspension obtained after milling (i.e. post-added), whereby a granular composition that has an excellent aggregation-suppressing effect and is finely milled to a submicron level can be prepared.

For example, when composition γ is prepared by wet milling, it is preferable that about 2 to 20 parts by mass of surfactant (B-2) is added based on 100 parts by mass of component (A) and wet milling is performed, and that surfactant (B-2) is post-added to the suspension after milling so that the total amount of surfactant (B-2) based on 100 parts by mass of component (A) in the composition is more than 20 parts by mass and 55 parts by mass or less. Compositions α and β can also be prepared by a method comprising such a post-addition step.

The content of surfactant (B-2) in the milled liquid during wet milling is, for example, preferably about 0.5 to 4% (w/w), and more preferably about 0.8 to 3% (w/w).

As described above, the composition of the present invention may further comprise sugar and/or sugar alcohol. The sugar and/or sugar alcohol may be added during wet milling, but is preferably added after wet milling. For example, when the composition of the present invention comprising sugar and/or sugar alcohol is prepared by wet milling, the sugar and/or sugar alcohol are not added during wet milling, but are preferably added to the suspension obtained after milling. By adjusting the amount of post-addition, the composition of the present invention comprising sugar and/or sugar alcohol in the above preferable range of the ratio of parts by mass can be easily prepared.

The suspension after milling thus obtained (in the case of a suspension prepared by a method comprising a post-addition step, it may be either before or after post-addition) can also be referred to as a dispersion composition (suspension composition) in which powder (granular composition) finely pulverized into a submicron level is dispersed in a medium (e.g., water). When the dispersion composition comprises surfactant (B-2), the content ratio of surfactant (B-2) is preferably 0.5 to 8% (w/w), and more preferably 1 to 7% (w/w). When the content ratio of surfactant (B-2) is within this range, the delamanid particle aggregation-suppressing effect in the milled dispersion is more preferably exhibited. For example, when the content ratio of surfactant (B-2) is within the above range, the particle aggregation-suppressing effect is more preferably exhibited even after standing for several days (e.g., 1, 2, or 3 days). As described above, it is preferable that the dispersion composition comprises a larger amount of surfactants (B-2) in order to enhance the particle aggregation-suppressing effect when the dispersion composition is dried or granulated to prepare a granular composition, and the granular composition is resuspended (redispersed) in a medium (e.g., water or a buffer).

In the present specification, the term "comprising" includes "consisting essentially of" and "consisting of." Further, the present invention includes all of any combinations of the constituent requirements described in the present specification.

In addition, the various characteristics (properties, structures, functions, etc.) described in each embodiment of the present invention described above may be combined in any way in specifying the subject matters included in the present invention. In other words, the present invention includes all the subject matters comprising all combinations of the combinable characteristics described in the present specification.

### Examples

The present invention is described in more detail below; however, the present invention is not limited to the following examples.

### Preparation 1: Preparation of submicron particles by wet-milling method

Compositions (suspensions) comprising delamanid, a polymer, dioctyl sodium sulfosuccinate (DOSS), and a sucrose fatty acid ester were prepared as described in Examples 1 to 3 below. In Examples 1 to 3, the type of polymer used was changed (Example 1: hydroxypropyl methylcellulose, Example 2: PVP, and Example 3: hydroxypropyl cellulose), and the other conditions were the same.

### Example 1: Wet-milled suspension (polymer: TC-5E, surfactants: DOSS and sucrose stearate ester)

2.0 g of delamanid (hammer-milled powder, Otsuka Pharmaceutical Co., Ltd.), 0.1 g of hydroxypropyl methylcellulose (product name: TC-5E, Shin-Etsu Chemical Co., Ltd.), 0.15 g of dioctyl sodium sulfosuccinate (product name: Docusate 100, Cytec Solvay Group), and 0.1 g of sucrose fatty acid ester (product name: Sucrose Fatty Acid Ester S-1670, Mitsubishi-Chemical Foods Corporation) were dispersed and dissolved in 7.65 g of water (delamanid was dispersed, and the other components were dissolved; hereinafter the same). The resultant was placed in a container together with 8 g of zirconia beads with a diameter of 0.1 mm, and milled in a rotation/revolution Cooling Nano Pulverizer (NP-100, Thinky Corporation) at a temperature of -20°C and a rotation speed of 1700 rpm, thereby obtaining a milled suspension.

### Example 2: Wet-milled suspension (polymer: PVP, surfactants: DOSS and sucrose stearate ester)

2.0 g of delamanid (hammer-milled powder, Otsuka Pharmaceutical Co., Ltd.), 0.1 g of polyvinylpyrrolidone (product name: PVP, BASF Japan Ltd.), 0.15 g of dioctyl sodium sulfosuccinate (product name: Docusate 100, Cytec Solvay Group), and 0.1 g of sucrose fatty acid ester (product name: Sucrose Fatty Acid Ester S-1670, Mitsubishi-Chemical Foods Corporation) were dispersed and dissolved in 7.65 g of water. The resultant was placed in a container together with 8 g of zirconia beads with a diameter of 0.1 mm, and milled in a rotation/revolution Cooling Nano Pulverizer (NP-100, Thinky Corporation) at a temperature of -20°C and a rotation speed of 1700 rpm, thereby obtaining a milled suspension.

### Example 3: Wet-milled suspension (polymer: hydroxypropyl cellulose, surfactant: DOSS and sucrose stearate ester)

2.0 g of delamanid (hammer-milled powder, Otsuka Pharmaceutical Co., Ltd.), 0.1 g of hydroxypropyl cellulose (product name: HPC-SSL, Nippon Soda Co., Ltd.), 0.15 g of dioctyl sodium sulfosuccinate (product name: Docusate 100, Cytec Solvay Group), and 0.1 g of sucrose fatty acid ester (product name: Sucrose Fatty Acid Ester S-1670, Mitsubishi-Chemical Foods Corporation) were dispersed and dissolved in 7.65 g of water. The resultant was placed in a container together with 8 g of zirconia beads with a diameter of 0.1 mm, and milled in a rotation/revolution Cooling Nano Pulverizer (NP-100, Thinky Corporation) at a temperature of -20°C and a rotation speed of 1700 rpm, thereby obtaining a milled suspension.

In Examples 1 to 3, the change in the average particle size (Z-average) of the delamanid particles was measured every 3 minutes from the start of milling. Specifically, it was measured using a particle size distribution measuring device (Zetasizer Nano ZS, Spectris Co., Ltd., Malvern Division) with dynamic light scattering as the measurement principle. Hereinafter, the delamanid average particle size (change) was measured in the same manner. The results are shown in Fig. 1a. Further, the beads were removed from the obtained milled suspension, the suspension was transferred to a beaker containing a stir bar and stored with stirring at the speed of 500 rpm for several days at room temperature. Then, the change in the average particle size (Z-average) of the delamanid particles was measured in the same manner. The results are shown in Fig. 1b. Figs. 1a and 1b also show the change in the average particle size (Z-average) of delamanid particles when wet milling was performed in the same manner, except that a polymer was not added and water was further added in an amount corresponding to the mass of the polymer (Comparative Example 1).

It was found that it was preferable to use polymers, and that it was particularly preferable to use, among them, hydroxypropyl cellulose and hydroxypropyl methylcellulose, from the viewpoint that the average particle size of the delamanid particles could be relatively reduced.

### Preparation 2: Preparation of submicron particles by wet-milling method

Compositions (suspensions) comprising delamanid particles, hydroxypropyl cellulose, and dioctyl sodium sulfosuccinate (DOSS) were prepared as described in Examples 4 to 6 below. In Examples 4 to 6, the type of hydroxypropyl cellulose (HPC) used was changed (Example 4: HPC-L, Example 5: HPC-SL, and Example 6: HPC-SSL), and the other conditions were the same. The characteristics of each HPC used are as follows.

HPC-L: The viscosity (mPa·s) of 20°C/2% aqueous solution is about 6 to 10, and the molecular weight measured by the GPC method is about 140000.
HPC-SL: The viscosity (mPa·s) of 20°C/2% aqueous solution is about 3 to 5.9, and the molecular weight measured by the GPC method is about 100000.
HPC-SSL: The viscosity (mPa·s) of 20°C/2% aqueous solution is about 2 to 2.9, and the molecular weight measured by the GPC method is about 40000.

In all of the above HPCs, the degree of substitution with propyl groups is 70 to 75%.

### Example 4: Wet-milled suspension (HPC-L, DOSS)

100 g of delamanid (hammer-milled powder, Otsuka Pharmaceutical Co., Ltd.), 12 g of hydroxypropyl cellulose (product name: HPC-L, Nippon Soda Co., Ltd.), and 5 g of dioctyl sodium sulfosuccinate (product name: Docusate 100, Cytec Solvay Group) were dispersed and dissolved in 883 g of water. The resultant was milled with 1700 g of zirconia beads with a diameter of 0.3 mm in a DYNO-MILL with a capacity of 0.6 L (Multi-Lab, Shinmaru Enterprises Corporation) at an agitator disk rotation speed of 10 m/sec, thereby obtaining a milled suspension.

### Example 5: Wet-milled suspension (HPC-SL, DOSS)

100 g of delamanid (hammer-milled powder, Otsuka Pharmaceutical Co., Ltd.), 12 g of hydroxypropyl cellulose (product name: HPC-SL, Nippon Soda Co., Ltd.), and 5 g of dioctyl sodium sulfosuccinate (product name: Docusate 100, Cytec Solvay Group) were dispersed and dissolved in 883 g of water. The resultant was milled with 1700 g of zirconia beads with a diameter of 0.3 mm in a DYNO-MILL with a capacity of 0.6 L (Multi-Lab, Shinmaru Enterprises Corporation) at an agitator disk rotation speed of 10 m/sec, thereby obtaining a milled suspension.

### Example 6: Wet-milled suspension (HPC-SSL, DOSS)

100 g of delamanid (hammer-milled powder, Otsuka Pharmaceutical Co., Ltd.), 12 g of hydroxypropyl cellulose (product name: HPC-SSL, Nippon Soda Co., Ltd.), and 5 g of dioctyl sodium sulfosuccinate (product name: Docusate 100, Cytec Solvay Group) were dispersed and dissolved in 883 g of water. The resultant was milled with 1700 g of zirconia beads with a diameter of 0.3 mm in a DYNO-MILL with a capacity of 0.6 L (Multi-Lab, Shinmaru Enterprises Corporation) at an agitator disk rotation speed of 10 m/sec, thereby obtaining a milled suspension.

In Examples 4 to 6, the change in the average particle size (Z-average) of the delamanid particles was measured every 5 minutes from the start of milling in the same manner as above. The results are shown in Fig. 2.

It was found that it was particularly preferable to use HPC-SL or HPC-SSL among the hydroxypropyl celluloses, from the viewpoint that the average particle size of the delamanid particles could be relatively reduced.

### Preparation of submicron particles by wet-milling method (preparation of Examples 7 to 24)

In the same manner as in Examples 4 to 6 above, according to the formulations shown in Table 1 below, delamanid, various polymers, various surfactants, and water were milled with 1700 g of zirconia beads with a diameter of 0.3 mm in a DYNO-MILL (Multi-Lab, Shinmaru Enterprises Corporation) with a capacity of 0.6 L at an agitator disk rotation speed of 10 m/sec, thereby preparing milled suspensions. Table 1 below also shows the formulations of Examples 4 to 6. The unit of the formulation of each component in Table 1 is grams (g).

Among the components shown in Table 1, the polyvinyl caprolactam-polyvinyl acetate-polyethylene glycol graft copolymer (Solplus) was purchased from BASF Japan Ltd. Further, the various sucrose fatty acid esters used were purchased from Mitsubishi-Chemical Foods Corporation. The characteristics of each sucrose fatty acid ester are as follows.
- Sucrose Fatty Acid Ester S-1670: sucrose stearate ester, HLB: about 16, monoester content: about 75%, di-tri-polyester content: about 25%
- Sucrose Fatty Acid Ester S-1170: sucrose stearate ester, HLB: about 11, monoester content: about 55%, di-tri-polyester content: about 45%
- Sucrose Fatty Acid Ester S-570: sucrose stearate ester, HLB: about 5, monoester content: about 30%, di-tri-polyester content: about 70%
- Sucrose Fatty Acid Ester 0-1570: sucrose oleate ester, HLB: about 15, monoester content: about 70%, di-tri-polyester content: about 30%

Polysorbate 80 and sucrose fatty acid esters (also referred to as "SE" in the tables and drawings) are nonionic surfactants, and DOSS and SLS are anionic surfactants.

Table 1 also shows the average particle size (Z-average) of the delamanid particles after milling. The examples labeled with "2L scale" in Table 1 indicate that milled suspensions were prepared by milling delamanid using double the amount of each component shown in Table 1.

Each composition was milled until the average particle size of the delamanid particles hardly changed even after the milling treatment.

**Table 1**

| | | Example 4 | Example 5 | Example 6 | Example 7 | Example 8 | Example 9 | Example 10 | Example 11 | Example 12 | Example 13 | |
|---|---|---|---|---|---|---|---|---|---|---|---|---|
| | Delamanid (hammer-miled powder) | 100 | 100 | 100 | 100 | 200 | 100 | 50 | 50 | 50 | 50 | |
| Polymer | Hydraxypropyl cellulose (HPC: HPC-L) | 12 | - | - | - | - | - | 6 | 6 | 6 | 6 | |
| | Hydraxypropyl cellulose (HPC: HPC-SL) | - | 12 | - | - | - | 12 | - | - | - | - | |
| | Hydraxypropyl cellulose (HPC: HPC-SSL) | - | - | 12 | - | 10 | - | - | - | - | - | |
| | Polyvinyl caprolactam-polyvinyl acetate-polyathytere glycol graft polymer (Sdplus) | - | - | - | 12 | - | 5 | - | - | - | - | |
| Surfactant | Diocty sodium sulfosuccinate (DOSS: Docusate 100) | 5 | 5 | 5 | 10 | 15 | 10 | 2.5 | 2.5 | 2.5 | - | |
| | Sucrose fatty acid ester (Sucrose Fatly Add Ester S-1670) | - | - | - | 5 | 10 | - | - | - | - | - | |
| | Sucrose fatty acid ester (Sucrose Fatty Acid Ester S-1170) | - | - | - | - | - | - | - | 2.5 | - | - | |
| | Sucrose fatty acid ester (Sucrose Fatly Add Ester S-570) | - | - | - | - | - | - | 2.5 | - | - | - | |
| | Sucrose fatty acid ester (Sucrose Fatty Add Ester 0-1570) | - | - | - | - | - | - | - | - | 2.5 | - | |
| | Sodium dodecyl sulfate (NIKKOL SLS) | - | - | - | - | - | - | - | - | - | 2.5 | |
| | Polysorbate 80(NIKKOL TO-10MV) | - | - | - | - | - | - | - | - | - | - | |
| | Water | 883 | 883 | 883 | 873 | 765 | 873 | 939 | 939 | 939 | 941.5 | |
| | Total | 1000 | 1000 | 1000 | 1000 | 1000 | 1000 | 1000 | 1000 | 1000 | 1000 | |
| | | | | | 2L scale | | | | | | | |
| | Average particle size (Z-average) after milling | 269 nm | 232 nm | 250 nm | 224 nm | 205 nm | 209 nm | 240 nm | 238 nm | 228 nm | 240 nm | |

| | | Example 14 | Example 15 | Example 16 | Example 17 | Example 18 | Example 19 | Example 20 | Example 21 | Example 22 | Example 23 | Example 24 |
|---|---|---|---|---|---|---|---|---|---|---|---|---|
| | Delamanid (hammer-millad powder) | 100 | 100 | 100 | 100 | 100 | 100 | 100 | 200 | 200 | 250 | 200 |
| Polymer | Hydraxypropyl cellulose (HPC: HPC-L) | 12 | 12 | 12 | - | - | - | - | - | - | - | - |
| | Hydraxypropyl cellulose (HPC: HPC-SL) | - | - | - | 17 | 12 | 7 | 12 | - | - | - | - |
| | Hydraxypropyl cellulose (HPC: HPC-SSL) | - | - | - | - | - | - | - | 24 | 24 | 30 | 20 |
| | Polyvinyl caprolactam-polyvinyl acetate-polyethylene glycol graft polymer (Solplus) | - | - | - | - | - | - | - | - | - | - | - |
| Surfactant | Dioctyl sodium sulfosuccinate (DOSS: Docusate 100) | - | - | 5 | 10 | 10 | 10 | - | 30 | 20 | 18.75 | 15 |
| | Sucrose fatty acid ester (Sucrose Fatty Add Ester S-1670) | - | - | - | 5 | 5 | 5 | 5 | - | 10 | 12.5 | 10 |
| | Sucrose fatty acid ester (Sucrose Fatty Add Ester S-1170) | - | 5 | 5 | - | - | - | - | - | - | - | - |
| | Sucrose fatty acid ester (Sucrose Fatty Add Ester S-570) | - | - | - | - | - | - | - | - | - | - | - |
| | Sucrose fatty acid ester (Sucrose Fatty Add Ester O-1570) | - | - | - | - | - | - | - | - | - | - | - |
| | Sodium dodecyl sulfate (NIKKOLSLS) | - | - | - | - | - | - | 10 | - | - | - | - |
| | Polysorbate 80 (NIKKOL TO-10MV) | 5 | - | - | - | - | - | - | - | - | - | - |
| | Water | 883 | 883 | 878 | 868 | 873 | 878 | 873 | 746 | 746 | 688.75 | 755 |
| | Total | 1000 | 1000 | 1000 | 1000 | 1000 | 1000 | 1000 | 1000 | 1000 | 1000 | 1000 |
| | | | | | 2L scale | 2L scale | 2L scale | 2L scale | 2L scale | 2L scale | 2L scale | 2L scale |
| | Average partide size (Z-average) after milling | 963 nm | 926 nm | 221 nm | 210 nm | 211 nm | 208 nm | 256 nm | 366 nm | 223 nm | 197 nm | 197 nm |

The change in the average particle size (Z-average) of the delamanid particles in Examples 4, 13, 14, 15, and 16 was measured in the same manner as above. The results are shown in Fig. 3a. In Fig. 3a, Example 16 is expressed as "DOSS + SE," Example 4 is expressed as "DOSS," Example 15 is expressed as "SE," Example 13 is expressed as "SLS," and Example 14 is expressed as "Polysorbate 80." From the viewpoint that the average particle size of the delamanid particles can be relatively reduced, Examples 16, 4, and 13 are preferable, Examples 16 and 4 are more preferable, and Example 16 is particularly preferable. Therefore, it was found that any anionic and nonionic surfactants could be used as the surfactants, that among them, DOSS was preferably used, and that DOSS and a sucrose fatty acid ester (sucrose stearate ester) were particularly preferably used in combination.

Moreover, the change in the average particle size (Z-average) of the delamanid particles in Example 18 (DOSS + SE) and Example 20 (SLS + SE) was measured in the same manner as above. The results are shown in Fig. 3b. Fig. 3b revealed that the combined use of DOSS and a sucrose fatty acid ester (sucrose stearate ester) was more preferable than the combined use of SLS and a sucrose fatty acid ester (sucrose stearate ester).

In addition, the change in the average particle size (Z-average) of the delamanid particles in Examples 21 and 22 was measured in the same manner as above. The results are shown in Fig. 4. Fig. 4 revealed that the combined use of DOSS and a sucrose fatty acid ester (sucrose stearate ester) (Example 22) was more preferable than the use of DOSS alone (Example 21).

### Drying 1: Drying of submicron particles after wet milling

In the milled suspensions obtained in Examples 4 to 6 above, mannitol was added and dissolved in an amount of 30 mass%, 50 mass%, or 100 mass% of delamanid in the milled suspensions. Alternatively, the milled suspension obtained in Example 4 was used as it is, without adding mannitol. The obtained suspensions were each spray-dried using a spray dryer (GB-22, Yamato Scientific Co., Ltd.) to obtain submicron powders. The spray conditions were an inlet temperature of 140°C, a spray speed of 10 g/min, and an air volume of 0.4 to 0.5 m³/min. The obtained powder samples were dried in a vacuum dryer (LCV-232, Tabai Espec Corp.) at 60°C for 24 hours to remove the remaining water. (Submicron powders obtained by adding mannitol to each of the milled suspensions of Examples 4, 5, and 6, followed by spray drying are referred to as "Example I-1," "Example 1-2," and "Example 1-3," respectively. Further, submicron powder obtained by directly spray-drying the milled suspension of Example 4 is referred to as "Example I-i.")

The obtained submicron powders were each evaluated for their redispersibility in water. Specifically, 2 mg of each submicron powder was added to 10 mL of water, the mixture was stirred with a vortex mixer (SI-0286, Scientific Industries, Inc.) to prepare a dispersion, and the average particle size (Z-average) of particles in the dispersion was measured. The measurement was carried out using a particle size distribution measuring device (Zetasizer Nano ZS, Spectris Co., Ltd., Malvern Division) with dynamic light scattering as the measurement principle.

The average particle size measurement results (Z-average) are shown in Table 2 below.

**Table 2**

| Ratio of mannitol added | Example 1-1 | Example I-2 | Example I-3 | Example I-i |
|---|---|---|---|---|
| 0% | - | - | - | 437 nm |
| 30% | 313 nm | 291 nm | 365 nm | - |
| 50% | 285 nm | 289 nm | 286 nm | - |
| 100% | 269 nm | 282 nm | 288 nm | - |

It was found that the examples in which mannitol was added were superior in redispersibility in water.

### Drying 2: Drying of submicron particles after wet milling

### Example II

In the milled suspension obtained in Example 8, mannitol in an amount of 60 mass% of delamanid in the milled suspension, and dioctyl sodium sulfosuccinate (DOSS) in an amount of 22.5 mass% of delamanid in the milled suspension were added and dissolved. The obtained suspension was spray-dried using a spray dryer (GB-22, Yamato Scientific Co., Ltd.) to obtain submicron powder. The spray conditions were an inlet temperature of 140°C, a spray speed of 10 g/min, and an air volume of 0.4 to 0.5 m³/min. The obtained powder sample was dried in a vacuum dryer (LCV-232, Tabai Espec Corp.) at 60°C for 24 hours to remove the remaining water. The obtained powder was used as Example II.

### Example III

In the milled suspension obtained in Example 13, mannitol was added and dissolved in an amount of 100 mass% of delamanid in the milled suspension. The obtained suspension was spray-dried using a spray dryer (GB-22, Yamato Scientific Co., Ltd.) to obtain submicron powder. The spray conditions were an inlet temperature of 140°C, a spray speed of 10 g/min, and an air volume of 0.4 to 0.5 m³/min. The obtained powder sample was dried in a vacuum dryer (LCV-232, Tabai Espec Corp.) at 60°C for 24 hours to remove the remaining water. The obtained powder was used as Example III.

### Example IV

In the milled suspension obtained in Example 7, mannitol was added and dissolved in an amount of 200 mass% of delamanid in the milled suspension. The obtained suspension was spray-dried using a spray dryer (Type ODT-8, Ohkawara Kakohki Co., Ltd.) to obtain submicron powder. The spray conditions were an inlet temperature of 90°C, a spray speed of 80 g/min, and an atomizer rotation speed of about 13000 rpm. The obtained powder sample was dried in a vacuum dryer (LCV-232, Tabai Espec Corp.) at 60°C for 24 hours to remove the remaining water. The obtained powder was used as Example IV.

### Example V

In the milled suspension obtained in Example 9, mannitol was added and dissolved in an amount of 100 mass% of delamanid in the milled suspension. The obtained suspension was spray-dried using a spray dryer (Type ODT-8, Ohkawara Kakohki Co., Ltd.) to obtain submicron powder. The spray conditions were an inlet temperature of 90°C, a spray speed of 80 g/min, and an atomizer rotation speed of about 13000 rpm. The obtained powder sample was dried in a vacuum dryer (LCV-232, Tabai Espec Corp.) at 60°C for 24 hours to remove the remaining water. The obtained powder was used as Example V.

### Examples VI, VII, and VIII

In the milled suspension obtained in Example 17, 18, or 19, mannitol was added and dissolved in an amount of 100 mass% of delamanid in the milled suspension. The obtained suspensions were each spray-dried using a spray dryer (Type ODT-8, Ohkawara Kakohki Co., Ltd.) to obtain submicron powders. The spray conditions were an inlet temperature of 90°C, a spray speed of 80 g/min, and an atomizer rotation speed of about 13000 rpm. The obtained powder sample was dried in a vacuum dryer (LCV-232, Tabai Espec Corp.) at 60°C for 24 hours to remove the remaining water.

The submicron particles obtained from the milled suspensions of Examples 17, 18, and 19 were used as Examples VI, VII, and VIII, respectively.

### Examples IX, X, and XI

In a milled suspension prepared in the same manner as in Example 18 (i.e., using different lots from Example 18), or in the milled suspension obtained in Example 20, mannitol was added and dissolved in an amount of 100 mass% of delamanid in the milled suspension. Further, in the milled suspension obtained in Example 22, mannitol in an amount of 90 mass% of delamanid in the milled suspension, and dioctyl sodium sulfosuccinate (DOSS) in an amount of 10 mass% of delamanid in the milled suspension were added and dissolved.

The obtained suspensions were each spray-dried using a spray dryer (Type ODT-8, Ohkawara Kakohki Co., Ltd.) to obtain submicron powders. The spray conditions were an inlet temperature of 100°C, a spray speed of 80 g/min, and an atomizer rotation speed of about 13000 rpm. The obtained powder sample was dried in a vacuum dryer (LCV-232, Tabai Espec Corp.) at 60°C for 24 hours to remove the remaining water.

The submicron particles obtained as described above from the milled suspension using different lots from Example 18 and from the milled suspensions of Examples 20 and 22 were used as Examples XI, IX, and X, respectively.

The redispersibility of the submicron powders of Examples II and III in water was evaluated. Further, the redispersibility of the submicron powders of Examples IV to XI in water or McIlvaine buffer with a pH of 5.0 was evaluated. Specifically, 2 mg of each submicron powder was added to 10 mL of water or McIlvaine buffer with a pH of 5.0, the mixture was stirred with a vortex mixer (SI-0286, Scientific Industries, Inc.) to prepare dispersions, and the average particle size (Z-average) of particles in the dispersions was measured. The measurement was carried out using a particle size distribution measuring device (Zetasizer Nano ZS, Spectris Co., Ltd., Malvern Division) with dynamic light scattering as the measurement principle. The results are shown in Table 3.

**Table 3**

| | Example II | Example III | Example IV | Example V | Example VI | Example VII | Example VIII | Example IX | Example X | Example XI |
|---|---|---|---|---|---|---|---|---|---|---|
| Milled suspension | Example 8 | Example 13 | Example 7 | Example 9 | Example 17 | Example 18 | Example 19 | Example 20 | Example 22 | Prepared as in Example 18 (different lots) |
| Mannitol (relative to delamanid in milledsuspension) (mass%) | 60 | 100 | 200 | 100 | 100 | 100 | 100 | 100 | 90 | 100 |
| DOSS (relative to delamanid in milled suspension) (mass%) | 22.5 | - | - | - | - | - | - | - | 10 | - |
| Average partide size (Z-average) after redispersion (test liquid: water) | 208 nm | 505 nm | 349 nm | 430 nm | 248 nm | 239 nm | 260 nm | 292 nm | 230 nm | 245 nm |
| Average partide size (Z-average) after redispersion (test liquid: pH 5.0 Mcllvaine) | - | - | 569 nm | 645 nm | 243 nm | 255 nm | 247 nm | 329 nm | 224 nm | 258 nm |

### Drying 3: Drying of submicron particles after wet milling

### Example XII

In a milled suspension obtained in the same manner as in Example 8 (using zirconia beads with a diameter of 0.2 mm; 4 L scale), mannitol was added and dissolved in an amount of 60 mass% of delamanid in the milled suspension. Further, dioctyl sodium sulfosuccinate was added and dissolved in an amount of 22.5 mass% of delamanid. Low-substituted hydroxypropyl cellulose (product name: LH-B1, Shin-Etsu Chemical Co., Ltd.) was used as nuclear particles in an amount of 150 mass% of delamanid, and the nuclear particles were coated with the obtained suspension and dried using a multiplex (MP-01, Powrex, Inc.) to obtain submicron powder (fluidized bed granulation). Coating was performed at an inlet temperature of 60 to 80°C at a spray speed of 3 to 15 g/min at an air flow rate of 0.3 to 0.5 m³/hr. The obtained powder was dried using the same multiplex at 80°C for 30 minutes. The obtained powder was used as Example XII.

The redispersibility of the submicron powder of Example XII in water was evaluated in the same manner as above. Specifically, 2 mg of each submicron powder was added to 10 mL of water, the mixture was stirred with a vortex mixer (SI-0286, Scientific Industries, Inc.) to prepare dispersions, and the average particle size (Z-average) of particles in the dispersions was measured. The measurement was carried out using a particle size distribution measuring device (Zetasizer Nano ZS, Spectris Co., Ltd., Malvern Division) with dynamic light scattering as the measurement principle. As a result, the measured average particle size was 224 nm.

### Example XIII

In the milled suspension of Example 23, dextrin was added and dissolved in an amount of 37.5 mass% of delamanid in the milled suspension. Further, sucrose was added and dissolved in an amount of 37.5 mass% of delamanid (the average particle size of the delamanid particles at this stage was 208 nm). Calcium silicate (product name: Florite R, Tomita Pharmaceutical Co., Ltd.) in an amount of 100 mass% of delamanid was used as nuclear particles, supplied into a high shear granulation device Vertical Granulator (VG-5, Powrex Co., Ltd.), and granulated at 384 rpm. The obtained suspension was added to the powder being blended, and the mixture was granulated. The obtained granulated powder was taken out and dried in a vacuum dryer at 60°C overnight. The obtained powder was used as Example XIII.

### Example XIV

In the milled suspension of Example 23, dextrin was added and dissolved in an amount of 37.5 mass% of delamanid in the milled suspension (the average particle size of the delamanid particles at this stage was 215 nm). Further, sucrose in an amount of 37.5 mass% of delamanid, and dioctyl sodium sulfosuccinate (DOSS) in an amount of 7 mass% of delamanid were added and dissolved. Calcium silicate (product name: Florite R, Tomita Pharmaceutical Co., Ltd.) in an amount of 100 mass% of delamanid was used as nuclear particles, supplied into a high shear granulation device Vertical Granulator (VG-5, Powrex Co., Ltd.), and granulated at 384 rpm. The obtained suspension was added to the powder being blended, and the mixture was granulated. The obtained granulated powder was taken out and dried in a vacuum dryer at 60°C overnight. The obtained powder was used as Example XIV.

### Example XV

In the milled suspension of Example 24, mannitol was added and dissolved in an amount of 75 mass% of delamanid in the milled suspension. Further, dioctyl sodium sulfosuccinate was added and dissolved in an amount of 7.5 mass% of delamanid (the average particle size of the delamanid particles at this stage was 243 nm). Low-substituted hydroxypropyl cellulose (product name: LH-B1, Shin-Etsu Chemical Co., Ltd.) was used as nuclear particles in an amount of 100 mass% of delamanid, and the nuclear particles were coated with the obtained suspension and dried using a multiplex (MP-01, Powrex, Inc.) to obtain submicron powder (fluidized bed granulation). Coating was performed at an inlet temperature of 60 to 80°C at an air flow rate of 0.3 to 0.5 m³/hr at a spray speed 3 to 15 g/min. The obtained powder was dried using the same multiplex at 80°C for 30 minutes. The obtained powder was used as Example XV.

The redispersibility of the submicron powders of Examples XIII to XV in water or McIlvaine buffer with a pH of 5.0 was evaluated in the same manner as above. Specifically, 2 mg of each submicron powder was added to 10 mL of water or McIlvaine buffer with a pH of 5.0, the mixture was stirred with a vortex mixer (SI-0286, Scientific Industries, Inc.) to prepare dispersions, and the average particle size (Z-average) of particles in the dispersions was measured. The measurement was carried out using a particle size distribution measuring device (Zetasizer Nano ZS, Spectris Co., Ltd., Malvern Division) with dynamic light scattering as the measurement principle.

The average particle size measurement results were as shown below. The average particle size of the submicron powder of Example XIII was 300 nm when it was dispersed in water, and 475 nm when dispersed in the McIlvaine buffer with a pH of 5.0. The average particle size of the submicron powder of Example XIV was 256 nm when it was dispersed in water, and 341 nm when dispersed in the McIlvaine buffer with a pH of 5.0. The average particle size of the submicron powder of Example XV was 240 nm when it was dispersed in water, and 244 nm when dispersed in the McIlvaine buffer with a pH of 5.0.

### Drying 4: Drying of submicron particles after wet milling

The submicron powders obtained in Examples VI, VII, and VIII were each spread on a plastic Petri dish and stored for 2 weeks at 60°C under closed conditions or at 40°C at a relative humidity (RH) of 75% under open conditions. After 2 weeks, the redispersibility in water or McIlvaine buffer with a pH of 5.0 was evaluated in the same manner as above. Specifically, 2 mg of submicron powder was added to 10 mL of water or McIlvaine buffer with a pH of 5.0, the mixture was stirred with a vortex mixer (SI-0286, Scientific Industries, Inc.) to prepare dispersions, and the average particle size (Z-average) of particles in the dispersions was measured. The measurement was carried out using a particle size distribution measuring device (Zetasizer Nano ZS, Spectris Co., Ltd., Malvern Division) with dynamic light scattering as the measurement principle.

The results are shown in Table 4 below. This table also shows the results of redispersibility examination performed during preparation of each submicron powder.

**Table 4**

| - Delamanid particles: average particle size (nm) | | | |
|---|---|---|---|
| Test liquid: water | | | |
| Formulation | Example VI | Example VII | Example VIII |
| Milled suspension | 210 | 211 | 208 |
| Dispersed immediately after preparation of powder | 248 | 239 | 260 |
| Dispersed after storage at 60°C for 2 weeks | 258 | 256 | 259 |
| Dispersed after storage at 40°C/75% RH for 2 weeks | 250 | 246 | 239 |

| Test liquid: pH 5.0 Mcllvaine | | | |
|---|---|---|---|
| Formulation | Example VI | Example VII | Example VIII |
| Milled suspension | 210 | 211 | 208 |
| Dispersed immediately after preparation of powder | 243 | 255 | 247 |
| Dispersed after storage at 60°C for 2 weeks | 260 | 257 | 252 |
| Dispersed after storage at 40°C/75% RH for 2 weeks | 254 | 253 | 256 |

### Examination of effect of submicron particles

A milled suspension was obtained in the same manner as in Example 18, except that zirconia beads with a diameter of 0.2 mm were used. Using the obtained milled suspension, mannitol was added in an amount of 100 mass% of delamanid in the suspension, followed by spray drying as in Example VII to obtain submicron powder (Example T-I). Further, submicron powder was obtained in the same manner as in Example 18 and Example VII (Example T-II).

The average particle size (Z-average) of delamanid particles measured by redispersing the powder of Example T-I in water or McIlvaine buffer with a pH of 5.0 was 216 nm or 222 nm, respectively. The average particle size (Z-average) of delamanid particles measured by redispersing the powder of Example T-II in water or McIlvaine buffer with a pH of 5.0 was 302 nm or 315 nm, respectively.

Gelatin capsules filled with the submicron powder (Example T-I or T-II) as 50 mg of delamanid were forcibly orally administered to male Beagle dogs (about 30 months old, weight: 8 to 12 kg, Nosan Beagle, Narc). Immediately after the administration, 40 mL of water was forcibly administered. About 50 g of pellet diet CD-5 (CLEA Japan, Inc.) was fed 30 minutes before the administration, and fasting was continued until the last blood sampling. In addition, the same examination was performed using commercially available tablets containing 50 mg of delamanid (Deltyba (registered trademark) tablets 50 mg) placed in gelatin capsules. Further, as a control, 50 mg of powder (D50: about 2 µm) obtained by milling delamanid with a jet mill was suspended in 40 mL of 1.0% hydroxypropyl methylcellulose (product name: TC-5E, Shin-Etsu Chemical Co., Ltd.) solution, and the resultant was orally administered.

Blood was collected before administration, and 1, 2, 4, 6, 8, 10, 12, and 24 hours after administration, and about 1 mL of blood was collected (n = 6). The obtained blood was centrifuged at 3000 rpm for 10 minutes to obtain serum. The delamanid concentration of the serum was measured by LC-MS. The results are shown in Fig. 5.

Further, the maximum serum concentration (Cₘₐₓ), the area under the serum concentration curve (AUC), the time to reach the maximum plasma concentration (Tₘₐₓ), and the average residence time (MRTₗₐₛₜ) were calculated from the obtained serum concentration changes. The results are shown in Table 5 below.

**Table 5**

| | Cₘₐₓ (ng/mL) | AUCₐₗₗ (ng/mL·hr) | Tₘₐₓ (hr) | MRTₗₐₛₜ (hr) |
|---|---|---|---|---|
| Deltyba tablets | 303.0 | 4350.7 | 7.2 | 10.9 |
| Jet-milled powder | 153.3 | 2399.9 | 5.7 | 10.7 |
| Submicron formulation (Example T-I) | 520.3 | 6574.3 | 6.7 | 12.0 |
| Submicron formulation (Example T-II) | 514.2 | 7870.7 | 8.7 | 11.9 |

### Formulation Examples 1 to 15

Of the components shown in Table 6, components other than magnesium stearate or sodium stearyl fumarate were mixed in a polyethylene bag, and then magnesium stearate or sodium stearyl fumarate was added and further mixed to obtain mixed powders. The unit for the amount of each component shown in Table 6 is parts by mass.

In Formulation Examples 1 and 2, the mixed powder was compressed at a compression force of 7 kN (Formulation Example 1) or 8 kN (Formulation Example 2) using a rotary tablet press (Clean Press, Kikusui Seisakusho Ltd.) equipped with an 11-mm-diameter compound cup-shaped punches, thereby preparing caplet tablets containing 100 mg of delamanid per tablet. In Formulation Examples 3 to 12, the mixed powder was compressed at a compression force of 12 kN using a rotary tablet press (Clean Press, Kikusui Seisakusho Ltd.) equipped with a caplet-shaped (16.0 × 9.6 mm) punches, thereby preparing caplet tablets containing 100 mg of delamanid per tablet. In Formulation Examples 13 to 15, the mixed powder was compressed at a compression force of 8 kN using a rotary tablet press (Clean Press, Kikusui Seisakusho Ltd.) equipped with a caplet-shaped (13.6 × 6.8 mm) punches, thereby preparing caplet tablets containing 50 mg of delamanid per tablet.

**Table 6**

| | Formulation Example | | | | | | | | | | | | | | |
|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 |
| Delamanid submicron powder Example XI | 227 | | | | | | | | | | | | | | |
| Delamanid submicron powder Example IX | | 227 | | | | | | | | | | | | | |
| Delamanid submicron powder Example XII | | | 350 | 350 | 350 | 350 | 350 | 350 | 350 | 350 | 350 | 350 | 175 | 175 | 175 |
| Granulated mannitol (product name: Granutol S, Freund Corporation) | 63 | 23 | | | | 165 | 165 | 115 | 115 | 65 | 190 | 215 | | | |
| Delta mannitd (product name: Parteck Delta M, Merck) | | | 165 | | | | | | | | | | 104 | | |
| Spray-dried lactose (product name: Super Tab 11SD, DFE Pharma) | | | | 165 | | | | | | | | | | 84 | |
| Granulated lactose (product name: Dilactose S, Freund Corporation) | | | | | 165 | | | | | | | | | | |
| Crystalline cellulose (product name: Ceolus UF-711, Asahi Kasei Corporation) | 50 | 50 | | | | | | | | | | | 15 | 15 | 15 |
| Carmellose (product name: NS-300, Gotoku Chemical Co., Ltd.) | 50 | 50 | | | | | | | 50 | 50 | | | | | |
| Sodium starch glycolate (product name: Primojel, DFE Pharma) | 50 | 50 | | | | | | | | | | | | | |
| Croscarmellose sodium (product name: Kiccolate, Asahi Kasei Corporation) | | | | | | | | | | 50 | | | | | |
| Crospovidone (product name: Kollidon CL-F, BASF Japan Ltd.) | 50 | 50 | 100 | 100 | 100 | 100 | | 50 | 50 | 50 | 75 | 50 | 50 | 50 | 50 |
| Crospovidone (product name: Kollidon CL-SF, BASF Japan Ltd.) | | | | | | | 100 | | | | | | | | |
| Low-substituted hydroxypropyl cellulose (product name: LH-11, Shin-Etsu Chemical Co.. Ltd.) | | | | | | | | 100 | 50 | 50 | | | | | 84 |
| Light anhydrous silicic acid (product name: Adsolider 101, Freund Corporation) | 5 | 5 | 5 | 5 | 5 | 5 | 5 | 5 | 5 | 5 | 5 | 5 | 3 | 3 | 3 |
| Magnesium stearate (Taihei Chemical Industrial Co., Ltd.) | 5 | 5 | | | | | | | | | | | | | |
| Sodium stearyl fumarate (product name: Pruv, Rettenmaier Japan Co., Ltd.) | | | 5 | 5 | 5 | 5 | 5 | 5 | 5 | 5 | 5 | 5 | 3 | 3 | 3 |

### Formulation Examples 16 to 19

### Formulation Example 16

Delamanid (produced by Otsuka Pharmaceutical Co., Ltd.): 25 mg
Hydroxypropyl methylcellulose phthalate (HP-50, produced by Shin-Etsu Chemical Co., Ltd.): 75 mg
Lactose hydrate (Dilactose S, produced by Freund Corporation): 360 mg
Crospovidone (Kollidon CL-F, produced by BASF Japan Ltd.): 25 mg
Light anhydrous silicic acid (Adsolider 101, produced by Freund Corporation): 5 mg
Calcium stearate (produced by Taihei Chemical Industrial Co., Ltd.): 7.5 mg

Tablets were produced so that one tablet contained each of the above components. Specifically, 1 g of delamanid and 3 g of hydroxypropyl methylcellulose phthalate were dissolved in 100 ml of methylene chloride-ethanol mixed solvent (weight ratio of methylene chloride to ethanol = 8:2). Then, the mixture was spray-dried using a spray dryer (GS-310, produced by Yamato Scientific Co., Ltd.), and the obtained spray-dried product was further dried using a vacuum dryer (LCV-323, produced by Tabai Espec Corp.) at 60°C for 12 hours or longer, thereby producing a composition in powder form. This composition was mixed with other components and compressed to produce tablets. These tablets may also contain a sweetener, a flavoring agent, and the like, if necessary.

### Formulation Example 17

Delamanid (produced by Otsuka Pharmaceutical Co., Ltd.): 25 mg
Hydroxypropyl methylcellulose phthalate (HP-50, produced by Shin-Etsu Chemical Co., Ltd.): 75 mg
Mannitol (Granutol S, produced by Freund Corporation): 360 mg
Crospovidone (Kollidon CL-F, produced by BASF Japan Ltd.): 25 mg
Light anhydrous silicic acid (Adsolider 101, produced by Freund Corporation): 5 mg
Calcium stearate (produced by Taihei Chemical Industrial Co., Ltd.): 7.5 mg

Tablets were produced so that one tablet contained each of the above components. Specifically, 1 g of delamanid and 3 g of hydroxypropyl methylcellulose phthalate were dissolved in 100 ml of methylene chloride-ethanol mixed solvent (weight ratio of methylene chloride to ethanol = 8:2). Then, the mixture was spray-dried using a spray dryer (GS-310, produced by Yamato Scientific Co., Ltd.), and the obtained spray-dried product was further dried using a vacuum dryer (LCV-323, produced by Tabai Espec Corp.) at 60°C for 12 hours or longer, thereby producing a composition in powder form. This composition was mixed with other components and compressed to produce tablets. These tablets may also contain a sweetener, a flavoring agent, and the like, if necessary.

### Formulation Example 18

Tablets were produced in the same manner as in Formulation Example 17, except that mannitol (Parteck Delta M, produced by Merck) was used in place of the mannitol (Granutol S, produced by Freund Corporation).

### Formulation Example 19

Tablets were produced in the same manner as in Formulation Example 17, except that in addition to delamanid and hydroxypropyl methylcellulose phthalate, 0.08 g of dl-a-tocopherol (produced by Wako Pure Chemical Industries, Ltd.) was dissolved in a methylene chloride-ethanol mixed solvent in the production of a composition in powder form.

## Claims

1. A composition comprising:
(A) delamanid particles, and
(B) a surface stabilizer.

2. A composition comprising:
(A) delamanid particles,
(B-1) a polymer, and
(B-2) a surfactant.

3. The composition according to claim 2, comprising at least hydroxypropyl cellulose as polymer (B-1).

4. The composition according to claim 2 or 3, comprising at least a nonionic surfactant and/or an anionic surfactant as surfactant (B-2).

5. The composition according to any one of claims 2 to 4, comprising (A) delamanid particles, (B-1) hydroxypropyl cellulose, and (B-2) a sucrose fatty acid ester and/or a diester of alkyl alcohol and sulfosuccinic acid or a salt thereof.

6. The composition according to any one of claims 2 to 5, comprising 2 to 20 parts by mass of component (B-1) and 2 to 55 parts by mass of component (B-2) based on 100 parts by mass of component (A).

7. The composition according to claim 6, comprising 2 to 15 parts by mass of sucrose fatty acid ester and 2 to 40 parts by mass of dioctyl sodium sulfosuccinate based on 100 parts by mass of component (A).

8. The composition according to any one of claims 2 to 7, further comprising sugar and/or sugar alcohol.

9. The composition according to claim 8, comprising 2 to 20 parts by mass of component (B-1), 2 to 55 parts by mass of component (B-2), and 30 to 200 parts by mass of sugar and/or sugar alcohol, based on 100 parts by mass of component (A).

10. The composition according to claim 8 or 9, comprising 2 to 15 parts by mass of sucrose fatty acid ester, 2 to 40 parts by mass of dioctyl sodium sulfosuccinate, and 30 to 200 parts by mass of mannitol, based on 100 parts by mass of component (A).

11. The composition according to any one of claims 1 to 10, which is a granular composition.

12. The composition according to any one of claims 1 to 11, wherein delamanid particles (A) have an average particle size of 350 nm or less.

13. A pharmaceutical oral solid formulation obtainable from the granular composition according to claim 11 or 12.

14. The pharmaceutical oral solid formulation according to claim 13, which is a tablet or capsule.
